# NEUE EUROPÄISCHE PATENTSCHRIFT

(11) **EP 2 767 174 B2**
(45) Veröffentlichungstag und Bekanntmachung des Hinweises auf die Entscheidung über den Einspruch: **01.07.2020**
(45) Hinweis auf die Patenterteilung: 14.12.2016
(21) Anmeldenummer: 13155556.7
(22) Anmeldetag: 16.02.2013
(51) Int. Cl.: A23L 33/00, A61K 31/16, A61K 31/164

(54) **Orale Zubereitungen**
Oral compositions
Compositions orales

(43) Veröffentlichungstag der Anmeldung: 20.08.2014
(73) Patentinhaber: Symrise AG, 37603 Holzminden (DE)
(72) Erfinder: Ley, Jakob, 37603 Holzminden (DE); Widder, Sabine, 37603 Holzminden (DE); Krammer, Gerhard, 37603 Holzminden (DE); Somoza, Veronika, 3400 Weidling (AT); Rohm, Barbara, 1090 Wien (AT); Zaunschirm, Mathias, 3552 Lengenfeld (AT)
(74) Vertreter: Eisenführ Speiser

(56) Entgegenhaltungen:
- EP-A1- 1 829 582
- EP-A1- 1 977 655
- EP-A1- 2 008 530
- EP-A1- 2 058 297
- EP-A1- 2 058 297
- EP-A1- 2 236 489
- EP-A1- 2 305 048
- EP-A1- 2 471 383
- WO-A1-2011/112067
- WO-A1-2011/112067
- WO-A2-2004/000787
- WO-A2-2004/043906
- WO-A2-2004/043906
- US-A1- 2008 050 500

## Beschreibung

### Gebiet der Erfindung

Die Erfindung befindet sich auf dem Gebiet Nahrungsmittel und Nahrungsergänzungsstoffe und betrifft eine Zubereitung mit einem Gehalt an mindestens einem Alkamid zur Bekämpfung von Übergewicht und den damit verbundenen negativen physischen und psychischen Faktoren.

### Stand der Technik

Das durch mangelnde Bewegung und/oder zu hohe Nahrungsaufnahme verursachte häufige Auftreten von andauerndem Übergewicht kann zu chronischen Krankheitsbildern wie Fettleibigkeit, Insulinresistenz, Lipidstoffwechselstörungen und/oder Bluthochdruck, deren schwerwiegenden Folgeerkrankungen Diabetes Typ II, Arteriosklerose, Herz- oder Hirninfarkt und damit letztendlich zum verfrühten Tod führen. Insbesondere ein hoher Gehalt an leicht verstoffwechselbaren Kohlenhydraten, Proteinen und vor allem Fetten in der Nahrung führt zur Bildung von Fettdepots und kann schließlich zu den oben genannten Problemen stark beitragen. Um die Aufnahme dieser hedonisch oft sehr bevorzugten Nahrungsmittelbestandteile, vor allem der Fette und süßen Kohlenhydrate (Zucker), zu begrenzen, werden oft deren Gehalte in kalorienreduzierten Lebensmitteln, den sogenannten "Light-Produkten", stark gesenkt und durch Ersatzstoffe (Verdicker für Fette, nicht-kalorische Süßstoffe statt der Zucker) ausgetauscht.

Beim Konsum von "Light-Produkten" kann es bei einem Produkt, dass aufgrund geschickter Formulierung einen vergleichbaren hedonischen Wert wie das hoch-energetische Originalprodukt besitzt, oft vorkommen, dass es in größeren Mengen konsumiert wird und es dann im ungünstigsten Fall sogar zu einer erhöhten Aufnahme kalorisch relevanter Nahrungsmittelbestandteile kommt und somit das Ziel verfehlt wird, die aufgenommene Kalorienmenge abzusenken.

Um einer erhöhten Aufnahme kalorisch relevanter Nahrungsmittelbestandteile entgegenzuwirken, besteht schon länger der Wunsch, Nahrungsbestandteile, insbesondere als sicher bewertete, bereits zugelassene und allgemein akzeptierte Aromastoffe zu finden, die das Hungergefühl und den natürlichen Appetit reduzieren und/oder das Sättigungsgefühl entsprechend erhöhen können. Es ist bekannt, dass durch Erhöhung der Dopamin- und der Serotonin-Ausschüttung in bestimmten Hirnarealen bei gleichzeitiger Exposition mit Nährstoffen durch die aufgenommenen oral konsumierbaren Produkte (insbesondere Lebensmittel) sowie durch Induktion von Leptin-Rezeptor- und Serotonin-Rezeptor-Proteinen der Appetit negativ und die Sättigung positiv beeinflusst werden können.

Neben Auswirkungen auf den Appetit und auf die Sättigung, kann auch die Stimmung des Konsumenten positiv beeinflusst werden. So sind bereits Lebensmittel bekannt, die Dopamin und Serotonin umfassen und eine stimmungsaufhellende Wirkung besitzen. Sofern Serotonin in ausreichenden Mengen im Hirn vorhanden ist, vermittelt es eine positive Stimmung, eine gute Konzentrationsfähigkeit und Optimismus. Niedrige Serotoninspiegel können hingegen zu Reizbarkeit, Schlafstörungen, mangelnder Konzentrationsfähigkeit und Depressionen führen. Dopamin beeinflusst Aufmerksamkeit, Freude sowie geistige Klarheit. Ein Dopaminmangel macht sich unter anderem durch Apathie, mangelnde Liebesfähigkeit sowie fehlendes Reuebewusstsein bemerkbar.

Eine Aufnahme von Serotonin und Dopamin über die Nahrung erhöht die Serotonin- bzw. Dopaminkonzentration im Blut und steht mit einiger Wahrscheinlichkeit auch für Wechselwirkungen mit Rezeptoren im Gehirn zur Verfügung. Um die Serotonin- bzw. Dopaminkonzentration im Gehirn zu erhöhen, ist es vorteilhaft, die Ausschüttung von Serotonin bzw. Dopamin im Hirn zu stimulieren.

Noch direkter kann der Aufnahme kalorisch relevanter Nahrungsmittelbestandteile entgegengewirkt werden, wenn deren Absorption vermindert wird, da dann z.B. die körpereigenen Reserven stärker beansprucht werden und letztendlich nicht nur körpereigene Fettspeicher abgebaut, sondern zudem die Anzahl der Fettspeicherzellen zumindest nicht gesteigert wird. Dabei ist es insbesondere hilfreich, der Absorption der langkettigen freien Fettsäuren (FFA) oder deren Langzeit-Akkumulation in Fettzellen als Triglyceride entgegenzuwirken. Eine Verringerung der Absorption von Fettsäuren im Dünndarm, wie sie bereits für Lipaseinhibitoren wir Olestra^{®} nachgewiesen wurde bei gleichzeitiger Steigerung des Abbaus endogener Fettreserven könnte somit zur Erzielung und zur Erhaltung eines gesunden Körpergewichtes beitragen und der Entstehung von Adipositas vorbeugen.

Die komplexe Aufgabe die der vorliegenden Erfindung zugrunde liegt, hat daher darin bestanden, einen vorzugsweise lebensmittelrechtlich zugelassenen Aromastoff oder einen pflanzlichen Extrakt, enthaltend diesen Aromastoff, zu finden, der gleichzeitig
- den Appetit reduziert
- beim Konsum ein Gefühl der Sättigung vermittelt und die
- die Stimmung aufhellt sowie in Summe
- die Energieaufnahme reduziert.

### Beschreibung der Erfindung

In einer ersten Ausführungsform betrifft der Gegenstand der Erfindung eine orale Zubereitung, enthaltend in Mengen von 0,001 µM bis 10 µM (0,002 mg/kg bis 2 mg/kg) mindestens ein Alkamid ausgewählt aus der in Anspruch 1 definierten Gruppe.

Überraschenderweise wurde gefunden, dass die erfindungsgemäßen Alkamide in der Lage sind, gleichzeitig bereits
- in einem Konzentrationsbereich von 0,001 µM bis 1 µM (ca. 0,002 mg/kg bis ca. 0,2 mg/kg) die Serotonin-Freisetzung in Neuronen um maximal bis zu ca. 170 % gegen die jeweilige Kontrolle anzuregen;
- in einer Konzentration von etwa 0,1 µM (ca. 0,2 mg/kg) die Dopamin-Freisetzung nach Acetylcholin-Stimulierung um bis zu ca. 170 % gegen die jeweilige Kontrolle anzuregen;
- in einer Konzentration 0,01 bis 10 µM (ca. 0,02 mg/kg bis ca. 2 mg/kg) die Aufnahme freier Fettsäuren in Adipocyten um 5 bis 10 % gegen die jeweilige Kontrolle zu senken; und
- in einer Konzentration 0,001 bis 1 µM (ca. 0,002 mg/kg bis ca. 0,2 mg/kg) die Langzeit-akkumulation von Triglyceriden in Adipocyten um 5 bis 7 % gegen die jeweilige Kontrolle zu senken.

Bevorzugtes Alkamid in den Zubereitungen ist 2E-Decensäure-N-isobutylamid.

Die jeweiligen Konzentrationen der Alkamide sind im Vergleich zu typischen Einsatzkonzentrationen, um einen Geschmackseffekt zu erzielen, gering, es ist also nicht mit stark wahrnehmbaren Tingling-Effekten zu rechnen.

### Alkamide

Die im Folgenden erfindungsgemäß zu verwendenden Alkamide sind insbesondere ausgewählt aus der Gruppe bestehend 2E,4E-Decadiensäure-N-isobutylamid (trans-Pellitorin); 2E,4Z-Decadiensäure-N-isobutylamid (cis-Pellitorin), 2E,4E-Decadiensäure-N-piperid (Achilleamid); 2E-Decensäure-N-isobutylamid; 2E,6Z,8E-Decatriensäure-N-isobutylamid (Spilanthol); 2E,4E-Dodecadiensäure-N-isobutylamid und 2E,4E-Tetradecadiensäure-N-isobutylamid.

Besonders bevorzugt sind die im folgenden erfindungsgemäß zu verwendenden Alkamide ausgewählt aus der Gruppe bestehend aus 2E,4E-Decadiensäure-N-isobutylamid (trans-Pellitorin, Formel 1), 2E-Decensäure-N-isobutylamid (Formel 2) und 2E,6Z,8E-Decatrien-säure-N-isobutylamid (Spilanthol, Formel 3)).

Die Verbindungen der Formeln (1), (2) und (3) sind in der folgenden Abbildung noch einmal zur Verdeutlichung aufgeführt:

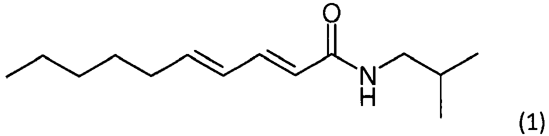

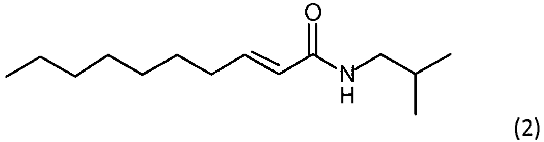

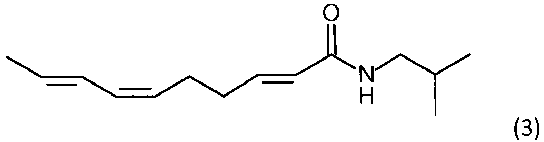

Alkamide, insbesondere 2E,4E-Decadiensäure-N-isobutylamid (trans-Pellitorin), 2E-Decensäure-N-isobutylamid und 2E,6Z,8E-Decatriensäure-N-isobutylamid (Spilanthol), sind bekannte Aromastoffe. Sie werden vor allem zur Erzielung eines sensorischen Kribbel-(englisch: Tingling)-Effekts (Bryant, B. P.; Mezine, I., Alkylamides that produce tingling paresthesia activate tactile and thermal trigeminal neurons. Brain Res. 1999, 842, 452-460) aber auch zur Förderung des Speichelflusses (z.B. wie in EP 1,562,893-B1 oder in WO 2005 044,778 beschrieben) sowie zur Maskierung von unangenehmen Geschmackseindrücken (wie z.B. in EP 2,058,297 oder EP 2,008,530-B1 beschrieben) verwendet.

In WO 2011 112,067 A1 wird ein Pflanzenextrakt (Nobesiti) aus *Piper sarmentosum* als Mittel zur Bekämpfung von Übergewicht beschrieben. Dabei ist in dem Extrakt neben Rutin, Flavonon, Sarmentin, Sarmentosin, Polyphenolen, weiteren Amiden und Glycosaponinen auch Pellitorin enthalten.

Für die Alkamide sind natürliche Quellen, vornehmlich Pflanzenextrakte bekannt und im ebenfalls Sinne der Erfindung einsetzbar. Für bevorzugte Alkamide sind pflanzliche Extrakte wie beispielsweise Alkamide enthaltender Pfefferextrakt (*Piper ssp.,* insbesondere *P. nigrum, P. hispidum, P. tuberculatum, P. longum, P. arboreum, P. futokadsura, P. guineense* oder *Piper nigrum, Var. muntok, P. aff. Pedicellatum*), Extrakte aus Zahnwehgras (englisch "toothache grass", *Ctenium aromaticum*), Extrakte aus Estragon (*Artemisia dracunculus*), Bertramwurzel-Extrakte (*Anacyclus ssp.,* insbesondere *Anacylcus pyrethrum* L.), Sonnenhutextrakte (*Echinaceae ssp.,* z.B. *E. angustifolia*), Extrakte aus Szechuan-Pfeffer (*Zanthoxylum* ssp., insbesondere *Zanthoxylum piperitum, Z. clava-herculin, Z. bungeanum, Z. zanthoxyloi*des), Spilanthes- oder Parakresseextrakte (*Spilanthes ssp.,* insbesondere *Spilanthes acmella*), Extrakte aus *Acmella ssp.* (z.B. *A.ciliata*), Extrakte aus *Achillea ssp.* (z.B. *Achillea wilsoniana*), Extrakte aus Fagara-Arten (*Fagara zanthoxyloides*), Extrakte aus *Heliopsis ssp.* (z.B. *H. longipes*), Extrakte aus *Cissampelos glaberrima,* Extrakte aus *Dinosperma erythrococca,* Extrakte aus der Rinde von *Esenbeckia alata* und Extrakte aus *Stauranthus perforatus* besonders geeignet.

Die pflanzlichen Extrakte enthaltend eines oder mehrere der Alkamide können aus den entsprechenden frischen oder getrockneten Pflanzen oder Pflanzenteilen, insbesondere aber aus weißen, grünen oder schwarzen Pfefferkörnern (*P. nigrum*), Stangenpfeffer (*P. longum*), Sonnenhutwurzeln, Bertramswurzel, Szechuan-Peffer, Pflanzenteilen der anderen *Zanthoxylum-Arten,* Pflanzenteilen der *Spilanthes-* oder *Acmella-Arten,* Pflanzenteilen der *Fagara-* oder *Heliopsis-Arten* gewonnen werden. Üblicherweise werden die getrockneten Pflanzenteile (z.B. frische oder getrocknete Wurzeln, Früchte, Samen, Rinde, Holz, Stängel, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise enzymatisch behandelt werden, mit Säure (z.B. unter Druck), mit sauren lonentauschern oder mit Wasserdampf behandelt werden, meist bei Drücken von 0,01 mbar bis 100 bar, vorzugsweise bei 1 mbar bis 20 bar, und/oder in einem für Nahrungs- und Genussmittel geeigneten Lösungsmittel aufgenommen werden. Bevorzugt sind Extrakte, in denen die Alkamide, insbesondere insbesondere 2E,4E-Decadiensäure-N-isobutylamid (trans-Pellitorin), 2E-Decensäure-N-isobutylamid und 2E,6Z,8E-Decatriensäure-N-isobutylamid (Spilanthol), den Hauptanteil bezogen auf die Gesamtmenge der Amid-artigen Verbindungen darstellen, bevorzugt 10 Gew.-%, besonders bevorzugt 20 Gew.-%, besonders bevorzugt 50 Gew.-%, ganz besonders bevorzugt 80 Gew.-% bezogen auf die Gesamtmenge der Amid-artigen Verbindungen darstellt.

Insbesondere für Nahrungs- und Genussmittel zur Extraktion geeignete Lösungsmittel sind Wasser, Ethanol, Methanol, 1,2-Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Die Alkamide können zusammen mit Hilfs- und Trägerstoffen konfektioniert werden. Bevorzugt für diesen Zweck sind Maltodextrin, Stärke, natürliche oder künstliche Polysaccharide und/oder Pflanzengummen wie modifizierte Stärken oder Gummi Arabicum, für die Aromakompositionen zugelassene Lösungsmittel wie z.B. Ethanol, 1,2-Propylenglycol, Wasser, Glycerin, Triacetin, pflanzliche Triglyceride, färbende Mittel, z.B. zugelassene Lebensmittelfarbstoffe, färbende Pflanzenextrakte, Stabilisatoren, Konservierungsstoffe, Antioxidantien, Viskositäts-beeinflussende Stoffe.

### Zubereitungen zur oralen Aufnahme

Im Sinne der vorliegenden Erfindung kann es sich bei den Zubereitungen um Nahrungsmittel, Futtermittel oder insbesondere um Nahrungsmittelergänzungsstoffe handeln, die die Alkamide bezogen auf die Gesamtmasse des oral konsumierbaren Produktes in Mengen von 2 mg/kg oder weniger, vorzugsweise in einer Konzentration von 0,20 mg/kg oder weniger, besonders bevorzugt in einer Konzentration von 0,10 mg/kg oder weniger enthalten. Vorzugsweise sind die Alkamide bezogen auf die Gesamtmasse des oral konsumierbaren Produktes in einer Konzentration von 0,002 mg/kg oder mehr vorhanden.

Bevorzugt ist ein erfindungsgemäß oral konsumierbares Produkt, bei dem es sich um eine flüssige oder feste Zubereitung, einschließlich einer Halbfertigware handelt.

Im Rahmen des vorliegenden Textes umfasst der Begriff "Lebensmittel" eine Vielzahl von Produkten. Unter den Begriff Lebensmittel fallen insbesondere Produkte, wie sie weiter unten im Zusammenhang mit erfindungsgemäßen Lebensmitteln diskutiert werden. Der Begriff umfasst insbesondere Produkte, die gemäß der VERORDNUNG (EG) Nr. 178/2002 DES EUROPÄISCHEN PARLAMENTS UND DES RATES vom 28. Januar 2002 Lebensmittel sind. Gemäß dieser Verordnung sind "Lebensmittel" alle Stoffe oder Erzeugnisse, die dazu bestimmt sind oder von denen nach vernünftigem Ermessen erwartet werden kann, dass sie in verarbeitetem, teilweise verarbeitetem oder unverarbeitetem Zustand von Menschen aufgenommen werden. Zu "Lebensmitteln" zählen auch Getränke, Kaugummi sowie alle Stoffe - einschließlich Wasser-, die dem Lebensmittel bei seiner Herstellung oder Ver- oder Bearbeitung absichtlich zugesetzt werden.

Der Begriff "Futtermittel" umfasst im Rahmen des vorliegenden Textes alle Formen von Tiernahrung. Eine Vielzahl der nachstehend genannten Lebensmittel kann auch als Futtermittel eingesetzt werden.

Es versteht sich, dass Lebensmittel oder Futtermittel durch den Zusatz von Stoffen oder Stoffzusammensetzungen, die als Mittel mit Eigenschaften zur Heilung oder zur Verhütung menschlicher oder tierischer Krankheiten bestimmt sind, in entsprechende Arzneimittel überführt werden können.

### Sprühgetrocknete Formulierungen

Speziell wenn es sich bei den erfindungsgemäßen Zubereitungen um Nahrungsmittelergänzungsstoffe handelt, stellen diese vorzugsweise um sprühgetrocknete Stoffe dar, die zum Verzehr geeignete Bestandteile, feste Trägerstoffe und ggf. bestimmte Aromastoffe bzw. Aromakompositionen umfassen. Dabei versteht es sich, dass diese Zusatzstoffe zum Verzehr geeignet sein müssen.

Vorteilhafte Trägerstoffe in diesen bevorzugten erfindungsgemäßen und vorzugsweise sprühgetrockneten Stoffmischungen sind Siliziumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliziumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Maisbasierende Stärken sowie Stärken aus Tapioka, Reis, Weizen oder Kartoffeln. Die Trägerstoffe können dabei auch als Fließhilfsmittel fungieren, wie beispielsweise Siliziumdioxid.

Die erfindungsgemäßen Stoffmischungen, die neben dem erfindungsgemäß zu verwendenden Alkamid oder mehreren Alkamiden noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Stoffmischungen, die mittels Sprühtrocknung hergestellt wurden, besitzen eine mittlere Partikelgröße im Bereich von 30 bis 300 µm und eine Restfeuchte von kleiner oder gleich 5 Gew.-%.

Ein weiterer Gegenstand der Erfindung betrifft daher ferner eine Stoffmischung, enthaltend
(a) eine wirksam Menge eines oder mehrerer der Alkamide,
(b) mindestens einen festen Trägerstoffen sowie gegebenenfalls
(c) mindestens einen Aromastoff.

Der optionale Aromastoff, der die Komponente (c) bildet, ist dabei vorzugsweise eine sensorisch wirksame Komponente und wird bevorzugt eingesetzt in einer Konzentration, die größer ist als sein Reizschwellenwert. Beispiele für in diesem Sinne geeignete Aromastoffe finden sich z.B. in H. Surburg, J. Panten, Common Fragrance and Flavor Materials, 5th. Ed., Wiley-VCH, Weinheim 2006. Die Aromastoffe können in Form von Aromakompositionen eingesetzt werden, die wiederum in Form von Reaktionsaromen (Maillard-Produkte) und/oder Extrakten bzw. ätherischen Ölen von Pflanzen oder Pflanzenteilen bzw. Fraktionen davon eingesetzt werden können.

### Verkapselte Formulierungen

Die Zubereitungen, speziell dann, wenn es sich um Ernährungsergänzungsstoffe handelt, können auch in verkapselter Form, d.h. als Makro- oder Mikrokapseln vorliegen.

Typische Makrokapseln werden durch Einschluss der Wirkstoffe gegebenenfalls in einer flüssigen Phase in Proteinen, wie insbesondere Gelatine erhalten und weisen dann Durchmesser im Bereich von etwa 0,5 bis 1 cm auf.

Unter den Begriffen "Mikrokapsel" oder "Nanokapsel" werden vom Fachmann hingegen sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapsein* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

### Lebensmittel

Um eine Reduktion des Körpergewichtes beim Konsumenten zu unterstützen, hat es sich als sinnvoll erwiesen, die potentielle kalorische Aufnahme zu begrenzen. Dies kann zum einen dadurch erfolgen, dass die erfindungsgemäße Verwendung des Alkamids oder einer Mischung der Alkamide in oral konsumierbaren Produkten (insbesondere Lebensmitteln, Futtermitteln und Arzneimitteln) ein Gefühl von Sättigung vermittelt und gleichzeitig den Appetit reduziert, sowie zusätzlich die Absorption von energieliefernden Nährstoffen aus dem Darm verringert. Dadurch wird der Konsument nicht nur dazu veranlasst, den Verzehr von energiehaltigen Produkten zu begrenzen, sondern ihm steht von der oral aufgenommenen Energiemenge durch eine verringerte Absorption auch weniger Nahrungsenergie zur Verfügung. Neben der durch die Alkamide verursachten geringeren Aufnahme und Akkumulation von Lipiden lässt die sich kalorische Aufnahme zusätzlich reduzieren, wenn erfindungsgemäße oral konsumierbare Produkte (insbesondere Lebensmittel, Futtermittel bzw. Arzneimittel) zum Verzehr angeboten werden, die ihrerseits bereits eine geringe Energiedichte aufweisen.

Ein bevorzugtes erfindungsgemäßes oral konsumierbares Produkt enthält nicht mehr als 200 kcal/100g des oral konsumierbaren Produktes, bevorzugt nicht mehr als 100 kcal/100g, besonders bevorzugt nicht mehr als 40 kcal/100g.

Bevorzugte erfindungsgemäße oral konsumierbare Produkte sind jegliche zum Verzehr geeignete der Ernährung, der Mundpflege oder dem Genuss dienende Zubereitungen oder Zusammensetzungen und sind regelmäßig Produkte, die dazu bestimmt sind, in die menschliche bzw. tierische Mundhöhle eingebracht zu werden, dort eine bestimmte Zeit zu verbleiben und anschließend entweder verzehrt (z.B. verzehrfertige Lebensmittel oder Futtermittel, siehe auch weiter unten) oder wieder aus der Mundhöhle entfernt zu werden (z.B. Kaugummis oder Mundpflegeprodukte oder medizinische Mundspülungen). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen oder Tier aufgenommen zu werden. Hierzu zählen auch Stoffe, die oral konsumierbaren Produkten (insbesondere Lebensmittel, Futtermittel und Arzneimittel) bei ihrer Herstellung, Verarbeitung oder Bearbeitung zugesetzt werden und dazu vorgesehen sind, in die menschliche oder tierische Mundhöhle eingebracht zu werden.

Zu den erfindungsgemäßen oral konsumierbaren Produkten zählen auch Stoffe, die dazu bestimmt sind, in unverändertem, zubereitetem oder verarbeitetem Zustand vom Menschen oder Tier geschluckt und dann verdaut zu werden; zu den erfindungsgemäßen oral konsumierbaren Produkten gehören insoweit auch Umhüllungen, Überzüge oder sonstige Umschließungen, die dazu bestimmt sind, mit verschluckt zu werden, oder bei denen ein Verschlucken vorauszusehen ist. Der Begriff "oral konsumierbares Produkt" umfasst verzehrfertige Lebensmittel und Futtermittel, d.h. Lebensmittel oder Futtermittel, die hinsichtlich der für den Geschmack maßgeblichen Substanzen bereits vollständig zusammengesetzt sind. Unter den Begriffen "verzehrfertiges Lebensmittel" oder "verzehrfertiges Futtermittel" fallen auch Getränke sowie feste oder halbfeste verzehrfertige Lebensmittel oder Futtermittel. Als Beispiele seien Tiefkühlprodukte genannt, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln oder Futtermitteln.

Bevorzugte erfindungsgemäße oral konsumierbare Produkte umfassen auch "Halbfertigwaren". Unter einer Halbfertigware ist im Zusammenhang des vorliegenden Textes ein oral konsumierbares Produkt zu verstehen, welches aufgrund eines sehr hohen Gehaltes an Aroma- und Geschmacksstoffen für die Verwendung als verzehrfertiges oral konsumierbares Produkt (insbesondere Lebensmittel oder Futtermittel) ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges oral konsumierbares Produkt (insbesondere Lebensmittel oder Futtermittel) überführt. Als Beispiel für Halbfertigwaren seien hier Tütensuppen, Backaromen und Puddingpulver genannt.

Zu erfindungsgemäßen oral konsumierbaren Produkten zählen auch "Mundpflegeprodukte". Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Aerosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

### A. Nahrungsmittelzubereitungen

Bevorzugt umfasst ein erfindungsgemäßes oral konsumierbares Produkt eine oder mehrere der Ernährung oder dem Genuss dienende Zubereitungen. Hierunter fallen insbesondere (kalorienreduzierte) Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Dragees, Hart- und Weichkaramellen, Kaugummi), nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegartes Gemüse, eingekochtes Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitungen zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen und als Nahrungsergänzungsmittel vorliegen.

### B. Kalorienreduzierte oder kalorienfreie Süßwaren

Besonders bevorzugt sind kalorienreduzierte Süßwaren (z.B. Müsliriegelprodukte, Fruchtgummi, Dragees, Hart- und Weichkaramellen, Kaugummi), nicht-alkoholische Getränke (z.B. Kakao, Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Trockenmilchpulver, Molke, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Süßstoffzubereitungen, -tabletten oder -sachets, sonstige Zubereitungen zum Süßen oder Weißen von Getränken oder anderen Nahrungsmitteln.

Insbesondere bevorzugt sind kalorienreduzierte oder kalorienfreie Süßwaren (z.B. Müsliriegelprodukte, Fruchtgummi, Dragees, Hartkaramellen, Kaugummi), nicht-alkoholische Getränke (z.B. Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder - freie Limonaden, isotonische Getränke, Nektare, Obst- und Gemüsesäfte, Frucht- oder Gemüsesaftzubereitungen), Instantgetränke (z.B. Instant-(Grün-, Schwarz, Roibos, Kräuter)Tee-Getränke), Getreideprodukte (z.B. zuckerarme oder -freie Frühstückscerealien, Müsliriegel), Milchprodukte (z.B. fettreduzierte oder fettfreie Milchgetränke, Joghurt, Kefir, Molke, Buttermilch), Produkte aus Sojaprotein oder anderen Sojabohnen-Fraktionen (z.B. Sojamilch und daraus gefertigte Produkte, Getränke, enthaltend isoliertes oder enzymatisch behandeltes Sojaprotein, Sojamehl enthaltende Getränke, Sojalecithin-haltige Zubereitungen, oder daraus gefertigte Produkte und Mischungen mit Fruchtzubereitungen und fakultativ Aromen) oder Süßstoffzubereitungen, -tabletten oder -sachets.

Die Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die Halbfertigwaren dienen in der Regel zur Herstellung von der Ernährung oder dem Genuss dienenden gebrauchs- oder verzehrfertigen Zubereitungen.

### C. Hilfs- und Zusatzstoffe

Weitere Bestandteile einer der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosin-monophosphat oder andere Stoffe wie Natriumglutamat oder 2-Phen-oxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße oral konsumierbare Produkte (insbesondere Lebensmittel, Futtermittel und Arzneimittel), z.B. solche in Form von Zubereitungen oder Halbfertigwaren, eine Aromakomposition, um den Geschmack und/oder den Geruch abzurunden und zu verfeinern. Eine Zubereitung kann als Bestandteile einen festen Trägerstoff und eine Aromakomposition umfassen. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, bevorzugt Protein[teil]hydrolysate mit hohem Arginin-Gehalt, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Kardamom, Muskat, Piment, Senf und Senf-Produkte), gebratenen, hefigen, gesotteen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

Die Energiedichte eines oral konsumierbaren Produktes (insbesondere Lebensmittels, Futtermittels oder Arzneimittels) lässt sich senken, indem energiereiche Inhaltsstoffe des oral konsumierbaren Produktes gegen Ersatzstoffe (z.B. kalorienarme Verdickungsmittel statt Fette, kalorienarme oder kalorienfreie Süßstoffe statt üblicher Zucker) ausgetauscht werden. Der bereits oben diskutierte Nachteil, dass der Konsument ein oral konsumierbares Produkt (insbesondere ein Lebensmittel) mit reduzierter Energiedichte in größeren Mengen konsumiert und es dann im ungünstigsten Fall sogar zu einer erhöhten Aufnahme kalorisch relevanter Nahrungsmittelbestandteile kommt, wird durch die Verwendung des Alkamids oder eines Gemischs von Alkamiden in oral konsumierbaren Lebensmitteln (insbesondere in Lebensmitteln) entgegengewirkt. In einem oral konsumierbaren Produkt enthaltenes Alkamids oder eines Gemischs von Alkamiden vermittelt ein vorzeitiges Gefühl von Sättigung und reduziert gleichzeitig den Appetit und die Resorption von Lipiden. Zudem hat es einen positiven Einfluss auf die Stimmung des Konsumenten, was sich ebenfalls positiv auswirkt. Vorzugsweise ist ein erfindungsgemäßes oral konsumierbares Produkt ausgewählt aus der Gruppe bestehend aus Süßwaren, vorzugsweise kalorienreduzierte bzw. kalorienfreie Süßwaren, vorzugsweise ausgewählt aus der Gruppe bestehend aus Müsliriegelprodukte, Fruchtgummi, Dragees, Hartkaramellen und Kaugummi,
- nicht-alkoholische Getränke, vorzugsweise ausgewählt aus der Gruppe bestehend aus Grüntee, Schwarztee, mit (Grün-, Schwarz-)Tee-Extrakten angereicherte (Grün-, Schwarz-)Tee-Getränke, Rooibos-Tee, andere Kräutertees, fruchthaltige zuckerarme oder -freie Limonaden, isotonische Getränke, Nektare, Obst- und Gemüsesäfte, Frucht- und Gemüsesaftzubereitungen,
- Instantgetränke, vorzugsweise ausgewählt aus der Gruppe bestehend aus Instant-(Grün-, Schwarz-, Rooibos-, Käuter-)Tee-Getränke,
- Getreideprodukte, vorzugsweise ausgewählt aus der Gruppe bestehend aus zuckerarmen und -freien Frühstückscerealien und Müsliriegeln,
- Milchprodukte, vorzugsweise ausgewählt aus der Gruppe bestehend aus fettreduzierten und fettfreien Milchgetränken, Joghurt, Kefir, Molke, Buttermilch und Eiscreme,
- Produkte aus Sojaprotein oder anderen Sojabohnen-Fraktionen, vorzugsweise ausgewählt aus der Gruppe bestehend aus Sojamilch, aus Sojamilch gefertigten Produkten, Getränken enthaltend isoliertes oder enzymatisch behandeltes Sojaportein, Sojamehl enthaltenden Getränken, Sojalecithin-haltigen Zubereitungen, aus Sojalecithin-haltigen Zubereitungen gefertigten Produkten und Mischungen mit Fruchtzubereitungen und ggf. Aromen,
- Süßstoffzubereitungen, -tabletten und -sachets
- Zuckerfreie Dragees,
- Eiscreme, mit oder ohne Bestandteile auf Basis von Milch, vorzugsweise zuckerfrei.

Bevorzugt enthält ein erfindungsgemäßes oral konsumierbares Produkt einen, zwei oder mehrere Süßstoffe und/oder ein, zwei oder mehrere Verdickungsmittel.
(i) **Süßstoffe.** Der Begriff "Süßstoffe" bezeichnet hierbei Stoffe mit einer relativen Süßkraft von zumindest 25, bezogen auf die Süßkraft von Saccharose (welches somit die Süßkraft 1 hat). Vorzugsweise sind in einem erfindungsgemäßen oral konsumierbaren Produkt (insbesondere Lebensmittel, Futtermittel oder Arzneimittel) (a) einzusetzende Süßstoffe nicht-kariogen und/oder besitzen einen Energiegehalt von maximal 5 kcal pro Gramm des oral konsumierbaren Produktes. Vorteilhafte Süßstoffe in einem bevorzugten erfindungsgemäßen oral konsumierbaren Produkt sind ausgewählt aus den folgenden Gruppen (a1) und (a2):
   (a1) natürlich vorkommende Süßstoffe, vorzugsweise ausgewählt aus der Gruppe bestehend aus
   (a1-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
   (a1-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
   (a1-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus*), Citrus-Extrakten und Extrakten aus *Lippia dulcis;*
   (a2) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen des Acesulfam, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.
(ii) **Verdickungsmittel.** Vorteilhafte Verdickungsmittel in einem bevorzugten erfindungsgemäßen oral konsumierbaren Produkt (insbesondere Lebensmittel, Futtermittel oder Arzneimittel) sind ausgewählt aus der Gruppe bestehend aus: vernetzte Polyacrylsäuren und deren Derivate, Polysaccharide und deren Derivate, wie Xanthangummi, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.
Erfindungsgemäß bevorzugt ist ein oral konsumierbares Produkt (insbesondere Lebensmittel oder Futtermittel), das durch Milchsäurebakterien verdickte Milch und/oder durch Milchsäurebakterien verdickte Sahne enthält und vorzugsweise ausgewählt ist aus der Gruppe bestehend aus oral konsumierbaren Produkten mit einem Fettgehalt von 4,0 Gew.-% oder weniger, bevorzugt von 1,5 Gew.-% oder weniger, besonders bevorzugt 0,5 Gew.-% oder weniger, jeweils bezogen auf das Gesamtgewicht des oral konsumierbaren Produktes, und/oder ausgewählt ist aus der Gruppe bestehend aus Joghurt, Kefir und Quark.
Vorzugsweise enthält das erfindungsgemäße oral konsumierbare Produkt (insbesondere Lebensmittel oder Futtermittel), das durch Milchsäurebakterien verdickte Milch und/oder durch Milchsäurebakterien verdickte Sahne enthält, einen Energiegehalt von nicht mehr als 150 kcal/100g des oral konsumierbaren Produktes, bevorzugt nicht mehr als 100 kcal/100g, besonders bevorzugt nicht mehr als 75 kcal/100g, besonders bevorzugt nicht mehr als 50 kcal/100g.
Ein bevorzugtes erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, umfasst zusätzlich Früchte und/oder Fruchtzubereitungen.
Besonders bevorzugt ist ein erfindungsgemäßes oral konsumierbares Produkt, das durch Milchsäurebakterien verdickte Milch und/oder durch Milchsäurebakterien verdickte Sahne enthält, wobei das oral konsumierbare Produkt (i) Zucker und/oder (ii) Verdickungsmittel und/oder (iii) Geliermittel und/oder (iv) Süßungsmittel und/oder (v) Aromen und/oder (vi) Konservierungsstoffe enthält.
"Zucker" ist im Rahmen des vorliegenden Textes (sofern nicht anders angegeben oder aus dem Kontext anders ersichtlich) der Sammelbegriff für alle süß schmeckenden Saccharide (Einfach- und Doppelzucker).
Vorteilhafterweise ist ein erfindungsgemäßes oral konsumierbares Produkt (insbesondere Lebensmittel oder Futtermittel), das durch Milchsäurebakterien verdickte Milch und/oder durch Milchsäurebakterien verdickte Sahne enthält, ein oral konsumierbares Produkt, das ein Probiotikum enthält, wobei das Probiotikum vorzugsweise ausgewählt ist aus der Gruppe bestehend aus Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) und Lactobacillus rhamnosus (ATCC 53013).

### D. Kaugummis

Besonders bevorzugt ist ein erfindungsgemäßes oral konsumierbares Produkt, das ein Kaugummi ist und eine Kaugummibase enthält. Die Kaugummibase ist dabei vorzugsweise ausgewählt aus der Gruppe bestehend aus "chewing gum" - oder "bubble gum" - Basen. Letztere sind weicher, damit sich damit auch Kaugummiblasen bilden lassen. Erfindungsgemäß bevorzugte Kaugummibasen umfassen neben traditionell eingesetzten natürlichen Harzen oder dem Naturlatex Chicle Elastomere wie Polyvinylacetate (PVA), Polyethylene, (nieder-oder mittel-molekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336, US 5,601,858 oder US 6,986,709 beschrieben. Daneben umfassen erfindungsgemäß bevorzugt einzusetzende Kaugummibasen vorzugsweise weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Erfindungsgemäße Kaugummis (insbesondere wie vorstehend offenbart) umfassen vorzugsweise Bestandteile wie Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist unter anderem die Kombination der Aromen mit weiteren Stoffen, die kühlende, wärmende und/oder mundwässerndernde Eigenschaften aufweisen.

### E. Getränke

In einer weiteren bevorzugten Ausführungsform der Erfindung stellt das oral konsumierbare Produkt ein Getränk dar, welches vorzugsweise einen Zuckergehalt von 30 g/100 mL Getränk oder weniger, bevorzugt von 15 g/100 mL oder weniger, besonders bevorzugt 5 g/100 mL oder weniger aufweist, besonders bevorzugt kein Zucker enthält und/oder wobei das Getränk kein Ethanol oder maximal 0,1 Volumenprozent Ethanol enthält, bezogen auf das Volumen des Getränkes.

Im Rahmen der vorliegenden Erfindung sind erfindungsgemäße oral konsumierbare Produkte weniger bevorzugt, bei denen es sich um Ethanol enthaltende Getränke handelt.

Kein Ethanol bedeutet in der vorliegenden Erfindung, dass kein Ethanol zugesetzt wird und dass die Zubereitung weniger als 0,1 Vol.-%, bevorzugt weniger als 0,01 Vol.-% und besonders bevorzugt keine messbare Menge an Ethanol enthält.

Besonders bevorzugt sind erfindungsgemäße oral konsumierbare Produkte (vorzugsweise Lebensmittel, Futtermittel oder Arzneimittel), wobei es sich um ein kohlensäurehaltiges Getränk oder um ein kohlensäurefreies Getränk handelt.

### Gewerbliche Anwendbarkeit

Weitere Gegenstände der vorliegenden Erfindung betreffen eines oder mehrere nicht-therapeutisches Verfahren
- zur Verminderung des Appetits, insbesondere auch zur Gewichtsabnahme,
- zur Erzeugung eines Sättigungsgefühls,
- zur Verringerung der kalorischen Resorption, insbesondere auch zur Reduzierung der Aufnahme und Akkumulation von Lipiden und Triglyceriden und/oder
- zur Stimmungsaufhellung bei dem man einem menschlichen oder tierischen Lebewesen eine Zubereitung oral verabreicht, die eine wirksame Menge der Zubereitung nach Anspruch 1 enthält.

Ebenfalls Gegenstand der Erfindung ist eine Verwendung wie in den Ansprüchen definiert
- zur Verminderung des Appetits, insbesondere auch zur Gewichtsabnahme,
- zur Erzeugung eines Sättigungsgefühls, und/oder
- zur Verringerung der kalorischen Resorption, insbesondere auch zur Reduzierung der Aufnahme und Akkumulation von Lipiden und Triglyceriden.

Bezüglich der bevorzugten Ausführungsformen wird voll umfänglich auf die oben bereits ausführlich beschriebenen Ausgestaltungsformen Bezug genommen, so dass sich Wiederholungen, speziell mit Hinblick auf die bevorzugten Alkamide und deren Einsatzmenge erübrigen.

### Beispiele

### Zellmodell

Als Zellmodell für die Ausschüttung der Neurotransmitter Dopamin und Serotonin werden humane Neuroblastomazellen (SH-SY5Y, ATCC Nummer CRL-2266) verwendet. Die Kultivierung erfolgt bei 37 °C und 5 % CO₂-Gehalt mit einer Mischung, die zu gleichen Teilen aus Eagle's Minimum Essential Medium (MEM) und F12 Medium (jeweils mit 10 % FBS und 1 % Penicillin / Streptomycin) besteht. Für die Messung der Dopamin-und Serotoninfreisetzung werden die Zellen mit Trypsin geerntet und nach einer Vitalitätsprüfung durch Trypanblaufärbung in definierter Zellzahl in 35 mm Zellkulturschalen ausgesät.

Als Zellmodell für die kurzfristige Aufnahme von freien Fettsäuren und die langfristige Lipidakkumulierung in Adipocyten werden murine Fibroblasten (3T3-L1, ATTC-Nummer CL-173) nach Ausdifferenzierung zu Adipocyten verwendet. 3T3-L1 Fibroblasten werden bei 37 °C und 5 % CO₂-Gehalt mit Dulbecco's Modified Eagle Medium (DMEM) mit 10 % FBS, 4 % L-Glutamin und 1 % Penicillin / Streptomycin (=Basalmedium) kultiviert. Die Ausdifferenzierung zu Adipocyten erfolgt zwei Tage nach Erreichen der Konfluenz durch Zusatz von 10 µg/L Insulin,1 µM Dexamethason und 0.5 mM Isobutylmethylxanthin in das Kulturmedium (=Differenzierungsmedium). Nach zwei Tagen Kultivierung in Differenzierungsmedium werden die Adipocyten für zwei weitere Tage durch Zusatz von 10 µg/L Insulin in das Kulturmedium (=Ausreifungsmedium) ausgreift. Nach drei bis fünf weiteren Tagen in Basalmedium sind die Adipocyten optimal ausdifferenziert und zeigen den für Adipocyten typischen Phänotyp durch Einlagerung von Lipidtröpfchen.

Als Zellmodell für die Aufnahme von freien Fettsäuren in den Dünndarm wird die humane Kolonzellline CaCo-2 (ATCC-Nummer HTB-37) nach Ausdifferenzierung zu einem Enterozyten-entsprechenden Phänotyp verwendet. CaCo-2-Zellen werden bei 37 °C und 5 % CO₂-Gehalt mit Dulbecco's Modified Eagle Medium (DMEM) mit 10 % FBS, 2 % L-Glutamin und 1 % Penicillin / Streptomycin kultiviert. Die Ausdifferenzierung zu Enterozyten erfolgt durch eine 18-tägige Kultivierung der Zellen nach Erreichen der Konfluenz.

### Beispiel 1

### Freisetzung von Serotonin in einem experimentellen Zellsystem mit Alkamiden

Nach 3-minütiger Stimulierung von 1,25 * 10⁶ humanen Neuroblastomazellen (SH-SY5Y) mit 300 µL Krebs-Ringer-HEPES Puffer, 0,1 % Ascorbinsäure, pH 6,2, mit oder ohne Zusatz eines Alkamids (trans-Pellitorin, Dihydropellitorin, Spilanthol, wobei bei Zusatz des Alkamids in Konzentrationen von 0,001 µM, , 0,1 µM 1 µM und 10 µM im Krebs-Ringer-HEPES Puffer eingestellt wird), wird der Serotoningehalt mit Hilfe eines enzymbasierten Nachweisverfahrens (Serotonin-ELISA sensitiv, DLD Diagnostica, Hamburg, Deutschland) ermittelt. Die Zellen werden mit einem natriumlaurylsarcosinathaltigem Puffer lysiert und der DNA-Gehalt mit Hilfe einer NanoQuant-Platte (Tecan, Mennendorf, Schweiz) zur Normalisierung der Serotoninfreisetzung ermittelt.

**Tabelle 1** zeigt den Einfluss von trans-Pellitorin auf die Serotoninfreistezung in SH-SY5Y Zellen. Eine Konzentration von 100 nM t-Pellitorin steigert die Serotonin-Freisetzung um 40%.

Die **Abbildungen 1a und 1b** zeigen die Serotoninausschüttung nach Inkubation mit t-Pellitorin (2) (Abbildung 1A) in den Konzentrationen 0,001 µM, 0,1 µM, 1 µM und 10 µM oder weiteren Alkamiden (Abbildung 1B: 1 = t-Pellitorin, 2 = Dihydropellitorin, 3 = Spilanthol)). Ergebnisse sind in % zur Kontrolle (Puffer mit 0,1% Ethanol) dargestellt. n=3, tR=2. Signifikante Unterschiede der Behandlungen zur Kontrolle wurden mit einer one-way ANOVA mit anschließendem Holm-Sidak post hoc-Test getestet und entsprechend folgendem Schema markiert: * p≤ 0,05; ** p≤ 0,01; ***p≤ 0,001.

**Tabelle 1**

| Serotonin-Freisetzung SH-SY5Y-Zellen nach Stimulierung mit verschiedenen Konzentrationen der Alkamide | | |
|---|---|---|
| **Testsubstanz** | **T/C [%]** | **Standardabweichung [%]** |
| EtOH Kontrolle (t-Pellitorin) | 100,0 | 7,93 |
| trans-Pellitorin 0,001 µM | 105,3 | 19,9 |
| trans-Pellitorin 0,1 µM | 140,2 | 25,9 |
| trans-Pellitorin 1 µM | 128,8 | 36,1 |
| trans-Pellitorin, 10 µM | 93,02 | 12,7 |
| EtOH Kontrolle (Dihydropellitorin) | 100,0 | 22,2 |
| Dihydropellitorin 0,001 µM | 140,9 | 16,7 |
| Dihydropellitorin 0,1 µM | 87,41 | 29,5 |
| Dihydropellitorin 1 µM | 124,3 | 31,1 |
| Dihydropellitorin 10 µM | 144,5 | 55,8 |
| EtOH Kontrolle (Spilanthol) | 100,0 | 10,5 |
| Spilanthol 0,001 µM | 105,6 | 19,3 |
| Spilanthol 0,1 µM | 129,9 | 21,7 |
| Spilanthol 1 µM | 103,8 | 7,62 |
| Spilanthol 10 µM | 91,77 | 2,24 |

### Beispiel 2

Freisetzung von Dopamin in einem experimentellen Zellsystem mittels trans-Pellitorin Methode zur Messung der Freisetzung von Dopamin:
Nach 3-minütiger Stimulierung von 1,25 * 10⁶ humanen Neuroblastomazellen (SH-SY5Y) mit 350 µL Krebs-Ringer-HEPES Puffer (1), pH 5, mit oder ohne Zusatz von trans-Pellitorin (2) in den Konzentrationen 0,001 µM, 0,1 µM, 1 µM und 10 µM wird der Überstand mit 1 N HCl angesäuert und der Dopamingehalt mit Hilfe eines enzymbasierten Nachweisverfahrens (Dopamin-ELISA, DLD Diagnostica, Hamburg, Deutschland) ermittelt. Die Zellen werden mit einem natriumlaurylsarcosinathaltigen Puffer lysiert und der DNA-Gehalt mit Hilfe einer NanoQuant-Platte (Tecan, Mennendorf, Schweiz) zur Normalisierung der Dopaminfreisetzung ermittelt. Bei einer Ethanolkontrolle (EtOH-Kontrolle) wird kein trans-Pellitorin zum Krebs-Ringer-HEPES Puffer hinzugefügt, sondern der Puffer wird mit Ethanol versetzt, sodass eine 0,1% ethanolische Krebs-Ringer-HEPES Puffer-Lösung resultiert.

**Tabelle 2** zeigt die Dopamin-Freisetzung durch SH-SY5Y-Zellen nach Stimulierung mit verschiedenen Konzentrationen des Alkamides t-Pellitorin.

Aus der **Abbildung 2** ist die Dopaminausschüttung nach Inkubation mit t-Pellitorin (2) in den Konzentrationen 0,001 µM, 0,1 µM, 1 µM und 10 µM zu entnehmen. Die Ergebnisse sind in % zur Kontrolle (Puffer mit 0,1% Ethanol) dargestellt. n=3, tR=2. Signifikante Unterschiede der Behandlungen zur Kontrolle wurden mit einer one-way ANOVA mit anschließendem Holm-Sidak post hoc-Test getestet und entsprechend folgendem Schema markiert: * p≤ 0,05; ** p.≤ 0,01; ***p≤ 0,001.

**Tabelle 2**

| Dopamin-Freisetzung durch SH-SY5Y-Zellen nach Stimulierung mit verschiedenen Konzentrationen des Alkamides t-Pellitorin. | | |
|---|---|---|
| **Testsubstanz** | **T/C [%]** | **Standardabweichung [%]** |
| EtOH Kontrolle | 100,0 | 6,22 |
| trans-Pellitorin 0,001 µM | 99,68 | 52,61 |
| trans-Pellitorin 0,1 µM | 182,7 | 54,78 |
| trans-Pellitorin 1 µM | 106,8 | 9,877 |
| trans-Pellitorin 10 µM | 127,3 | 63,15 |

Aus Tabelle 2 und Abbildung 2 geht der starke Einfluss geringer Konzentrationen von trans-Pellitorin auf die Dopaminfreisetzung hervor. Bereits eine Konzentration von 100 nM des trans-Pellitorins führt zu einer 80 %igen Steigerung der Dopaminfreisetzung durch SH-SY5Y-Zellen

### Beispiel 3

### Reduktion der Fettsäureaufnahme in 3T3-L1-Zellen

Methode zur Bestimmung der Fettsäureaufnahme in 3T3-L1-Zellen:
Nach Ausdifferenzierung der 3T3-L1 Fibroblasten zu Adipocyten werden 65000 Zellen pro Vertiefung in eine 96-well Platte ausgestreut und die Zellen bei 37 °C vier Stunden mit Basalmedium (DMEM + 10% fötalem Rinderserum) und anschließend eine Stunde mit serumfreiem Medium inkubiert. Die Zellen werden mit 0,1 µM, 1 µM und 10 µM t-Pellitorin 30 Minuten vorbehandelt und die Aufnahme von freien Fettsäuren in die Zellen mit Hilfe der fluoreszenzmarkierten Fettsäure BODIPY ^{®} (QBT Fatty Acid Uptake Coroporation, Deutschland) an einem Fluoreszenz-Microplatten-Lesegerät (Tecan, Mennendorf, Schweiz) alle 20 Sekunden über einen Zeitraum von 60 Minuten gemessen.

**Tabelle 3** zeigt die Fettsäureaufnahme durch 3T3-L1 Zellen nach Stimulierung mit verschiedenen Konzentrationen des Alkamides t-Pellitorin

Aus der **Abbildung 3** geht die Fettsäureaufnahme nach 30-minütiger Vorinkubation mit unterschiedlichen Konzentrationen von trans-Pellitorin in den Konzentrationen 0,1 µM, 1 µM und 10 µM hervor. Ergebnisse sind als Fläche unter der Kurve nach 60 minütiger Messung im Vergleich zur Kontrolle (1, Puffer mit 0,1% Ethanol) dargestellt. n=3, tR=2-3. Signifikante Unterschiede der Behandlungen zur Kontrolle wurden mit einer one-way ANOVA mit anschließendem Holm-Sidak post hoc-Test getestet und. und entsprechend folgendem Schema markiert: * p≤ 0,05; ** p.≤ 0,01; ***p≤ 0,001

**Tabelle 3**

| Fettsäureaufnahme durch 3T3-L1 Zellen nach Stimulierung mit verschiedenen Konzentrationen des Alkamides t-Pellitorin | | |
|---|---|---|
| **Testsubstanz** | **T/C [%]** | **Standardabweichung [%]** |
| Ethanol Kontrolle | 100,0 | 5,12 |
| trans-Pellitorin 0,1 µM | 93,84 | 4,97 |
| trans-Pellitorin 1 µM | 96,63 | 2,58 |
| trans-Pellitorin 10 µM | 91,95 | 8,12 |

Die vorliegenden Daten zeigen, dass eine Vorinkubation mit 10 µM t-Pellitorin zu einer Reduktion der Aufnahme an freien Fettsäuren um 9 % in Adipocyten führt.

### Beispiel 4

### Reduktion der Langzeit-Lipidakkumulation in 3T3-L1-Zellen

Methode zur Bestimmung der Langzeit-Lipidakkumulation in 3T3-L1-Zellen:
Nach Ausdifferenzierung der 3T3-L1 Fibroblasten zu Adipocyten werden die Zellen 10 Tage mit trans-Pellitorin in den Konzentrationen 0,001 µM, µM 0,1 µM, 1 µM und 10 µM und einer finalen Konzentration von 10 µg/L Insulin behandelt. Die Zellen werden anschließend mit 10 %igem Paraformaldehyd fixiert, und die intrazellulären Lipidtropfen mit einer 2.1 % igen Oil-Red O-Lösungen in einem Isopropanol/Wasser (60/40)-Gemisch angefärbt. Farbstoffreste werden durch Waschen mit doppeltdestilliertem Wasser entfernt. Anschließend wird der Farbstoff Oil-Red O mit 100 % Isoporpanol herausgelöst und Absorption der Oil-Red O-Lösung, die sich proportional zu den Lipidtropfen der Zellen verhält, an einem Mikroplatten-Lesegerät bestimmt. (Tecan, Mennendorf, Schweiz) gemessen.

**Tabelle 4** zeigt die Lipidakkumulierung in 3T3-L1-Zellen nach Stimulierung mit verschiedenen Konzentrationen des Alkamides t-Pellitorin.

Aus der **Abbildung 4** ist die Lipidakkumulierung nach Inkubation mit unterschiedlichen Konzentrationen von trans-Pellitorin (2) in den Konzentrationen 0,001 µM, 0,1 µM, 1 µM und 10 µM zu entnehmen. Die Ergebnisse sind in % im Vergleich zur Kontrolle (1, Puffer 0,1% Ethanol) dargestellt. n=3, tR=1-4. Signifikante Unterschiede der Behandlungen zur Kontrolle wurden mit einer one-way ANOVA mit anschließendem Holm-Sidak post hoc-Test getestet und entsprechend folgendem Schema markiert: * p≤ 0,05; ** p.≤ 0,01; ***p≤ 0,001

**Tabelle 4**

| Lipidakkumulierung in 3T3-L1-Zellen nach Stimulierung mit verschiedenen Konzentrationen des Alkamides t-Pellitorin. | | |
|---|---|---|
| **Testsubstanz** | **T/C [%]** | **Standardabweichung [%]** |
| Ethanol Kontrolle | 100,0 | 0,93 |
| trans-Pellitorin 0,001 µM | 94,60 | 8,32 |
| trans-Pellitorin 0,1 µM | 92,94 | 3,28 |
| trans-Pellitorin 1 µM | 93,46 | 5,86 |

### Beispiel 5

### Reduktion der Fettsäureaufnahme in differenzierten CaCo-2-Zellen

Nach Ausdifferenzierung der CaCo-2 Zellen zu einem Enterozyten-Phänotyp werden die Zellen eine Stunde mit serumfreiem Medium inkubiert. Anschließend werden die Zellen mit 0,1 µM, 1 µM und 10 µM t-Pellitorin 30 Minuten vorbehandelt und die Aufnahme von freien Fettsäuren in die Zellen mit Hilfe der fluoreszenzmarkierten Fettsäure BODIPY^{®} (QBT Fatty Acid Uptake Corporation, Deutschland) an einem Fluoreszenz-Microplatten-Lesegerät (Tecan, Mennendorf, Schweiz) alle 20 Sekunden über einen Zeitraum von 60 Minuten gemessen.

**Tabelle 5** zeigt die Fettsäureaufnahme in differenzierte CaCo-2 nach Stimulierung mit verschiedenen Konzentrationen des Alkamides t-Pellitorin.

Aus der **Abbildung 5** ist die Fettsäureaufnahme nach 30-minütiger Vorinkubation mit unterschiedlichen Konzentrationen von trans-Pellitorin (2) in den Konzentrationen 0,1 µM, 1 µM, 10 µM und 100 µM zu entnehmen. Die Ergebnisse sind als Fläche unter der Kurve nach 60 minütiger Messung im Vergleich zur Kontrolle (1, Puffer mit 0,1% Ethanol) dargestellt. n=3, tR=2-3. Signifikante Unterschiede der Behandlungen zur Kontrolle wurden mit einer one-way ANOVA mit anschließendem Holm-Sidak post hoc-Test getestet und entsprechend folgendem Schema markiert: * p≤ 0,05; ** p.≤ 0,01; ***p≤ 0,001

**Tabelle 5**

| Fettsäureaufnahme in differnzierte CaCo-2 nach Stimulierung mit verschiedenen Konzentrationen des Alkamides t-Pellitorin. | | |
|---|---|---|
| **Testsubstanz** | **T/C [%]** | **Standardabweichung [%]** |
| Ethanol Kontrolle | 100.0 | 5.01 |
| trans-Pellitorin 0,1 µM | 107.8 | 7.84 |
| trans-Pellitorin 1 µM | 98.7 | 3.58 |
| trans-Pellitorin 10 µM | 97.8 | 6.81 |
| trans-Pellitorin 100 µM | 86.0 | 6.05 |

Die vorliegenden Daten zeigen, dass eine 30-minütige Vorinkubation mit 100 µM t-Pellitorin zu einer Reduktion der Aufnahme an freien Fettsäuren um 14 % in einem Enterozyten-Modell führt.

### Anwendungsbeispiele

Die vorliegende Erfindung wird im Folgenden durch Beispiele zur Anwendung im Rahmen einer erfindungsgemäßen Verwendung erläutert. Alle Mengenangaben verstehen sich dabei sofern nicht anders angegeben auf Gew.-%.

**Tabelle I**

| Erfrischungsgetränke (zuckerhaltig, kalorienreduziert, kalorienfrei) | | | | | | | |
|---|---|---|---|---|---|---|---|
| **Inhaltsstoffe** | **A** | **B** | **C** | **D** | **E** | **F** | **G** |
| Zucker(Sucrose) | 10 | 10 | 7 | - | - | 8 | 7 |
| Glucose/Fructose-Sirup aus Mais, enthaltend 55 Gew.-% Fructose | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Coffein | - | - | 0,01 | - | - | - | - |
| Zitronenaroma | 0,1 | 0,05 | - | 0,1 | 0,1 | 0,1 | 0,1 |
| Limonenaroma | - | 0,05 | - | - | - | - | - |
| Getränke-Emulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Extrakt aus Rubus suavissimus, enthaltend 5 Gew.-% Rubusosid bez. auf das Gesamtgewicht des Extraktes | - | - | - | - | - | 0,01 | - |
| Homoeriodictyol-Natriumsalz | - | - | 0,005 | 0,005 | - | - | - |
| trans-Pellitorin | 0,000002 | 0,00005 | - | 0,00001 | 0,000001 | 0,0001 | 0,00005 |
| Spilanthol | - | 0,00005 | 0,00005 | - | 0,000001 | - | - |
| Wasser | Ad 100 | | | | | | |

Die Zutaten wurden in der angegebenen Reihenfolge gemischt und mit Wasser auf 100 % aufgefüllt. Die Mischungen werden in Glasflaschen gefüllt und carbonisiert.

**Tabelle II**

| Verwendung in einem Kaugummi | | | |
|---|---|---|---|
| **Komponente** | **Inhaltsstoff** | **A** | **B** |
| **A** | Kaugummibase, Company "Jagum T" | 30,4899 | 30,49999 |
| B | Sorbit, pulverisiert | 39,00 | 39,00 |
| | Isomalt^{®} (Palatinit GmbH) | 9,50 | 9,50 |
| | Xylit | 2,00 | 2,00 |
| | Mannit | 3,00 | 3,00 |
| | Aspartam^{®} | 0,10 | 0,10 |
| | Acesulfam^{®} K | 0,10 | 0,10 |
| | Emulgum^{®} (Colloides Naturels, Inc.) | 0,30 | 0,30 |
| C | Sorbitol, 70% | 14,00 | 14,00 |
| | Glycerin | 1,00 | 1,00 |
| D | Pfefferminzaroma | 0,5 | 0,5 |
| | trans-Pellitorin | 0,00002 | 0,0002 |
| | Eriodictyol | 0,0100 | - |

Teile A bis D werden gemischt und intensiv geknetet. Die Rohmasse kann z.B. in Form von dünnen Streifen zu verzehrsfertigen Kaugummis verarbeitet werden.

**Tabelle III**

| Verwendung in Hartkaramellen | | | | |
|---|---|---|---|---|
| **Inhaltsstoffe** | **A** | **B** | **C** | **D** |
| Zucker | 74,50 | - | - | - |
| Palatinit, Typ M | - | 74,00 | 75,50 | 75,00 |
| Zitronensäure | 0,5 | 1,0 | 0,5 | - |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | 0,040 |
| Balansin A nach [SY317] | - | 0,005 | 0,010 | 0,005 |
| Hesperetin | - | 0,001 | - | 0,001 |
| Phloretin | - | 0,002 | - | - |
| trans-Pellitorin | 0,0002 | 0,00001 | 0,000002 | 0,0002 |
| Spilanthol | - | 0,00001 | - | - |
| Wasser | ad 100 | ad 100 | ad 100 | ad 100 |

Palatinit bzw. der Zucker wurden gegebenenfalls nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt.

**Tabelle IV**

| Fettarme Joghurts | | | | |
|---|---|---|---|---|
| **Inhaltsstoff** | **A** | **B** | **C** | **D** |
| Sucrose | 10 | 8 | 6 | - |
| Rebaudiosid A 98 % | - | - | - | 0,050 |
| Extrakt aus Rubus suavissimus, enthaltend 5 Gew.-% Rubusosid bezogen auf das Gesamtgewicht des Extraktes, z.B. von Plant-Extrakt | - | 0,010 | 0,010 | - |
| Hesperetin | - | 0,001 | 0,001 | 0,002 |
| Phloretin | - | - | 0,002 | 0,002 |
| Homoeriodictyol-Natriumsalz | - | - | - | 0,005 |
| trans-Pellitorin | 0,00002 | 0,0001 | 0,000002 | 0,0001 |
| Joghurt, 0,1 % Fett | zu 100 % auffüllen | | | |

Die Inhaltsstoffe wurden gemischt und auf 5°C gekühlt.

**Tabelle V**

| Fruchtgummis | | |
|---|---|---|
| **Inhaltsstoffe** | **A** | **B** |
| Saccharose | 34,50 | 8,20 |
| Glucosesirup, DE 40 | 31,89 | 30,09 |
| Iso Syrup C* Tru Sweet 01750 (Cerestar GmbH) | 1, 50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | |
| Kirscharoma, enthaltend 1 Gew.-% Hesperetin 2 und 0,3 Gew.-% Phloretin bezogen auf das Aroma | - | 0,10 |
| trans-Pellitorin | 0,0002 | 0,00002 |
| Spilanthol | 0,00001 | - |
| Wasser | ad 100 | ad 100 |

Polydextrose ist ein nicht süß schmeckendes Polysaccharid mit niedrigem Brennwert.

## Patentansprüche

1. Zubereitungen zur oralen Aufnahme, enthaltend in Mengen von 0,001 µM bis 10 µM (0,002 mg/kg bis 2 mg/kg) mindestens ein Alkamid ausgewählt aus der Gruppe, die gebildet wird von 2Z,4Z-Decadiensäure-N-isobutylamid; 2Z,4E-Decadiensäure-N-isobutylamid; 2E,4E-Decadiensäure-N-([2S]-2-methylbutyl)-amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E-Decensäure-N-isobutylamid; 2E,6Z,8E-Decatriensäure-N-([2S]-2-methylbutyl)-amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (α-Sanshool); 2E,6Z-8E,10E-Dodecatetraensäure-N-(2-hydroxy-2-methylpropyl)amid (α-Hydroxysanshool); 2E,6E,8E,10E-Dodecatetraensäure-N-(2-hydroxy-2-methylpropyl)amid (γ-Hydroxysanshool); 2E,4E-8Z,10E,12E-Tetradecapentaensäure-N-(2-hydroxy-2-methyl-propyl)amid (γ-Hydroxy-sanshool); 2E,4E,8E,10E,12E-Tetradecapentaensäure-N-(2-hydroxy-2-methyl-propyl)-amid (γ-Hydroxyisosanshool); 2E,4E,8Z,10E,12E-Tetradecapentaensäure-N-(2-methyl-2-propenyl)amid (γ-Dehydrosanshool); 2E,4E,8Z,10E,12E-Tetradecaentaensäure-N-(2-methylpropyl)amid (γ-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E-Tetradecadiensäure-N-(2-hydroxy-2-methylpropyl)amid (Tetrahydrobungeanool).

2. Zubereitungen nach Anspruch 1, **dadurch gekennzeichnet, dass** die zu verwendeten Alkamide aus der Gruppe bestehend aus 2E-Decensäure-N-isobutylamid ausgewählt sind.

3. Zubereitungen nach den Ansprüchen 1 und/oder 2, **dadurch gekennzeichnet, dass** es sich um Nahrungsmittel, Futtermittel oder Nahrungsmittelergänzungsmittel handelt.

4. Zubereitungen nach mindestens einem der Ansprüche 1 bis 3, **dadurch gekennzeichnet, dass** sie in sprühgetrockneter Form vorliegen.

5. Zubereitungen nach mindestens einem der Ansprüche 1 bis 3, **dadurch gekennzeichnet, dass** sie in verkapselter Form vorliegen.

6. Zubereitungen nach mindestens einem der Ansprüche 1 bis 5, **dadurch gekennzeichnet, dass** sie weiterhin Süßstoffe und/oder Verdickungsmittel enthalten.

7. Nicht-therapeutisches Verfahren zur Verminderung des Appetits, bei dem man einem menschlichen oder tierischen Lebewesen eine Zubereitung nach Anspruch 1 oral verabreicht.

8. Nicht-therapeutisches Verfahren zur Erzeugung eines Sättigungsgefühls, bei dem man einem menschlichen oder tierischen Lebewesen eine Zubereitung nach Anspruch 1 oral verabreicht.

9. Nicht-therapeutisches Verfahren zur Verringerung der kalorischen Resorption, bei dem man einem menschlichen oder tierischen Lebewesen eine Zubereitung nach Anspruch 1 oral verabreicht.

10. Nicht-therapeutisches Verfahren zur Stimmungsaufhellung, bei dem man einem menschlichen Lebewesen eine Zubereitung nach Anspruch 1 oral verabreicht.

11. Verwendung von Zubereitungen zur oralen Aufnahme, enthaltend in Mengen von 0,001 µM bis 10 µM (0,002 mg/kg bis 2 mg/kg) mindestens ein Alkamid ausgewählt aus der Gruppe, die gebildet wird von 2E,4E-Decadiensäure-N-isobutylamid (trans-Pellitorin); 2E,4Z-Decadiensäure-N-isobutylamid (cis-Pellitorin); 2Z,4Z-Decadiensäure-N-isobutylamid; 2Z,4E-Decadiensäure-N-isobutylamid; 2E,4E-Decadiensäure-N-([2S]-2-methylbutyl)-amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E-Decensäure-N-isobutylamid; 2E,6Z,8E-Decatriensäure-N-isobutylamid (Spilanthol); 2E,6Z,8E-Decatriensäure-N-([2S]-2-methylbutyl)-amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (a-Sanshool); 2E,6Z-8E,10E-Dodecatetraensäure-N-(2-hydroxy-2-methylpropyl)amid (α-Hydroxysanshool); 2E,6E,8E,10E-Dodecatetraensäure-N-(2-hydroxy-2-methylpropyl)amid (γ-Hydroxysanshool); 2E,4E-8Z,10E,12E-Tetradecapentaensäure-N-(2-hydroxy-2-methyl-propyl)amid (γ-Hydroxysanshool); 2E,4E,8E,10E,12E-Tetradecapentaensäure-N-(2-hydroxy-2-methylpropyl)-amid (γ-Hydroxyisosanshool); 2E,4E,8Z,10E,12E-Tetradecapentaensäure-N-(2-methyl-2-propenyl)amid (γ-Dehydrosanshool); 2E,4E,8Z,10E,12E-Tetradecaentaensäure-N-(2-methy)propyf)amid (γ-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobun-geanool) und 2E,4E-Tetradecadiensäure-N-(2-hydroxy-2-methylpropyl)amid (Tetrahydrobungeanool) als oral aufzunehmende Mittel zur Verminderung des Appetits.

12. Verwendung von Zubereitungen wie in Anspruch 11 definiert als oral aufzunehmende Mittel zur Erzeugung eines Sättigungsgefühls.

13. Verwendung von Zubereitungen wie in Anspruch 11 definiert als oral aufzunehmende Mittel zur Verringerung der kalorischen Resorption.

## Claims

1. Compositions for oral ingestion, containing in amounts of 0.001 µM to 10 µM (0.002 mg/kg to 2 mg/kg) at least one alkamide selected from the group consisting of 2Z,4Z-decadienoic acid-N-isobutylamide; 2Z,4E-decadienoic acid-N-isobutylamide; 2E,4E-decadienoic acid-N-([2S]-2-methylbutyl)amide; 2E,4E-Decadienoic acid-N-([2S]-2-methylbutyl)amide; 2E,4E-decadienoic acid-N-([2R]-2-methylbutylamide); 2E,4Z-decadienoic acid-N-(2-methylbutyl)amide; 2E,4E-decadienoic acid-N-piperidide (achilleamide); 2E-decenoic acid-N-isobutylamide; 2E,6Z,8E-decatrienoic acid-N-([2S]-2-methylbutyl)-amide (homospilanthol); 2E,6Z,8E-Decatrienoic acid-N-([2R]-2-methylbutyl)amide; 2E-decen-4-inoic acid-N-isobutylamide; 2Z-decen-4-inoic acid-N-isobutylamide; 2E,6Z,8E,10E-dodecatetraenoic acid-N-(2-methylpropyl)amide (a-sanshool); 2E,6Z-8E,10E-dodecatetraenoic acid-N-(2-hydroxy-2-methylpropyl)amide (a-hydroxysanshool); 2E,6E,8E,10E-dodecatetraenoic acid-N-(2-hydroxy-2-methylpropyl)amide (γ-hydroxysanshool); 2E,4E-8Z,10E,12E-tetradecapentaenoic acid-N-(2-hydroxy-2-methyl-propyl)amide (γ-hydroxysanshool); 2E,4E,8E,10E,12E-tetradecapentaenoic acid-N-(2-hydroxy-2-methyl-propyl)-amide (γ-hydroxyisosanshool); 2E,4E,8Z,10E,12E-tetradecapentaenoic acid-N-(2-methyl-2-propenyl)amide (γ-dehydrosanshool); 2E,4E,8Z,10E, 12E-tetradecapentaenoic acid-N-(2-methylpropyl)amide (γ-sanshool); 2E,4E,8Z,11Z-tetradecatetraenoic acid-N-(2-hydroxy-2-methylpropyl)amide (bungeanool); 2E,4E,8Z,11E-tetradecatetraenoic acid-N-(2-hydroxy-2-methylpropyl)amide (isobungeanool); 2E,4E,8Z-tetradecatrienoic acid-N-(2-hydroxy-2-methylpropyl)amide (dihydrobungeanool) and 2E,4E-tetradecadienoic acid-N-(2-hydroxy-2-methylpropyl)amide (tetrahydrobungeanool).

2. Compositions according to claim 1, **characterised in that** the alkamides to be used are selected from the group consisting of 2E-decenoic acid-N-isobutylamide.

3. Compositions according to claim 1 and/or 2, **characterised in that** they are a food, feed or dietary supplement.

4. Compositions according to at least one of the claims 1 to 3, **characterised in that** they are present in spray-dried form.

5. Compositions according to at least one of the claims 1 to 3, **characterised in that** they are present in encapsulated form.

6. Compositions according to at least one of the claims 1 to 5, **characterised in that** they further contain sweeteners and/or thickeners.

7. Non-therapeutic process for the reduction of appetite, wherein a composition according to claim 1 is orally administered to a human or animal subject.

8. Non-therapeutic process for the creation of a feeling of satiety, wherein a composition according to claim 1 is orally administered to a human or animal subject.

9. Non-therapeutic process for the reduction of caloric resorption, wherein a composition according to claim 1 is orally administered to a human or animal subject.

10. Non-therapeutic process for mood-enhancing purposes, wherein a composition according to claim 1 is orally administered to a human subject.

11. Use of compositions for oral ingestion, containing in amounts of 0.001 µM to 10 µM (0.002 mg/kg to 2 mg/kg) at least one alkamide selected from the group consisting of 2E,4E-decadienoic acid-N-isobutylamide (trans-pellitorine); 2E,4Z-decadienoic acid-N-isobutylamide (cis-pellitorine); 2Z,4Z-decadienoic acid-N-isobutylamide; 2Z,4E-decadienoic acid-N-isobutylamide; 2E,4E-decadienoic acid-N-([2S]-2-methylbutyl)amide; 2E,4E-decadienoic acid-N-([2S]-2-methylbutyl)amide; 2E,4E-decadienoic acid-N-([2R]-2-methylbutylamide); 2E,4Z-decadienoic acid-N-(2-methylbutyl)amide; 2E,4E-decadienoic acid-N-piperidide (achilleamide); 2E-decenoic acid-N-isobutylamide; 2E,6Z,8E-decatrienoic acid-N-isobutylamide (spilanthol); 2E,6Z,8E-decatrienoic acid-N-([2S]-2-methylbutyl)-amide (homospilanthol); 2E,6Z,8E-decatrienoic acid-N-([2R]-2-methylbutyl)amide; 2E-decen-4-inoic acid-N-isobutylamide; 2Z-decen-4-inoic acid-N-isobutylamide; 2E,6Z,8E,10E-dodecatetraenoic acid-N-(2-methylpropyl)amide (α-sanshool); 2E,6Z-8E,10E-dodecatetraenoic acid-N-(2-hydroxy-2-methylpropyl)amide (a-hydroxysanshool); 2E,6E,8E,10E-dodecatetraenoic acid-N-(2-hydroxy-2-methylpropyl)amide (γ-hydroxysanshool); 2E,4E-8Z,10E,12E-tetradecapentaenoic acid-N-(2-hydroxy-2-methyl-propyl)amide (γ-hydroxysanshool); 2E,4E,8E,10E,12E-tetradecapentaenoic acid-N-(2-hydroxy-2-methylpropyl)amide (γ-hydroxyisosanshool); 2E,4E,8Z,10E,12E-tetradecapentaenoic acid-N-(2-methyl-2-propenyl)amide (γ-dehydrosanshool); 2E,4E,8Z,10E,12E-tetradecapentaenoic acid-N-(2-methylpropyl)amide (γ-sanshool); 2E,4E,8Z,11Z-tetradecatetraenoic acid-N-(2-hydroxy-2-methylpropyl)amide (bungeanool); 2E,4E,8Z,11E-tetradecatetraenoic acid-N-(2-hydroxy-2-methylpropyl)amide (isobungeanool); 2E,4E,8Z-tetradecatrienoic acid-N-(2-hydroxy-2-methylpropyl)amide (dihydrobungeanool) and 2E,4E-tetradecadienoic acid-N-(2-hydroxy-2-methylpropyl)amide (tetrahydrobungeanool) as means to be orally ingested to reduce appetite.

12. Use of compositions as defined in claim 11 as means to be orally ingested to create a feeling of satiety.

13. Use of compositions as defined in claim 11 as means to be orally ingested to reduce caloric resorption.

## Revendications

1. Préparations pour l'ingestion par voie orale, contenant, en des quantités de 0,001 µM à 10 µM (0,002 mg/kg à 2 mg/kg), au moins un alcamide choisi dans le groupe qui est constitué par le N-isobutylamide de l'acide 2Z,4Z-décadiénoïque ; le N-isobutylamide de l'acide 2Z,4E-décadiénoïque ; le N-([2S]-2-méthylbutyl)-amide de l'acide 2E,4E-décadiénoïque ; le N-([2S]-2-méthylbutyl)-amide de l'acide 2E,4E-décadiénoïque ; le N-([2R]-2-méthylbutylamide) de l'acide 2E,4E-décadiénoïque ; le N-(2-méthylbutyl)amide de l'acide 2E,4Z-décadiénoïque ; le N-pipéride de l'acide 2E,4E-décadiénoïque (amide d'Achille) ; le N-isobutylamide de l'acide 2E-décénoïque ; le N-([2S]-2-méthylbutyl)-amide de l'acide 2E,6Z,8E-décatriénoïque (homospilanthol) ; le N-([2R]-2-méthylbutyl)amide de l'acide 2E,6Z,8E-décatriénoïque; le N-isobutylamide de l'acide 2E-décén-4-ynoïque ; le N-isobutylamide de l'acide 2Z-décén-4-ynoïque ; le N-(2-méthylpropyl) amide de l'acide 2E,6Z,8E,10E-dodécatétraénoïque (a-sanshool) ; le N-(2-hydroxy-2-méthylpropyl)amide de l'acide 2E,6Z,8E,10E-dodécatétraénoïque (a-hydroxysanshool) ; le N-(2-hydroxy-2-méthylpropyl)amide de l'acide 2E,6E,8E,10E-dodécatétraénoïque (y-hydroxysanshool) ; le N-(2-hydroxy-2-méthylpropyl)-amide de l'acide 2E,4E,8Z,10E,12E-tétradécapentaénoïque (y-hydroxy-sanshool) ; le N-(2-hydroxy-2-méthylpropyl)-amide de l'acide 2E,4E,8E,10E,12E-tétradécapentaénoïque (γ-hydroxyisosanshool) ; le N-(2-méthyle-2-propényle)-amide de l'acide 2E,4E,8Z,10E,12E-tétradécapentaénoïque (y-déshydrosanshool) ; le N-(2-méthylpropyl)amide de l'acide 2E,4E,8Z,10E,12E-tétradécapentaénoïque (y-sanshool) ; le N-(2-hydroxy-2-méthylpropyl)amide de l'acide 2E,4E,8Z,11Z-tétradécatétraénoïque (bungeanool) ; le N-(2-hydroxy-2-méthylpropyl)amide de l'acide 2E,4E,8Z,11E-tétradécatétraénoïque (isobungeanool) ; le N-(2-hydroxy-2-méthylpropyl)amide de l'acide 2E,4E,8Z-tétradécatriénoïque (dihydrobungeanool) et le N-(2-hydroxy-2-méthylpropyl)amide de l'acide 2E,4E-tétradécadiénoïque (tétrahydrobungeanool).

2. Préparations selon la revendication 1, **caractérisées par le fait que** les alcamides à utiliser sont choisis dans le groupe constitué par le N-isobutylamide de l'acide 2E-décénoïque.

3. Préparations selon les revendications 1 et/ou 2, **caractérisées par le fait qu'**il s'agit de denrées alimentaires, d'aliments pour animaux ou de compléments alimentaires.

4. Préparations selon au moins l'une quelconque des revendications 1 à 3, **caractérisées par le fait qu'**elles se présentent sous forme séchée par pulvérisation.

5. Préparations selon au moins l'une quelconque des revendications 1 à 3, **caractérisées par le fait qu'**elles se présentent sous forme encapsulée.

6. Préparations selon au moins l'une quelconque des revendications 1 à 5, **caractérisées par le fait qu'**elles contiennent en outre des édulcorants et/ou des épaississants.

7. Procédé non thérapeutique destiné à diminuer l'appétit, dans lequel on administre par voie orale une préparation selon la revendication 1 à un être vivant humain ou animal.

8. Procédé non thérapeutique destiné à obtenir une sensation de satiété, dans lequel on administre par voie orale une préparation selon la revendication 1 à un être vivant humain ou animal.

9. Procédé non thérapeutique destiné à diminuer la résorption calorique, dans lequel on administre par voie orale une préparation selon la revendication 1 à un être vivant humain ou animal.

10. Procédé non thérapeutique destiné à améliorer l'humeur, dans lequel on administre par voie orale une préparation selon la revendication 1 à un être vivant humain.

11. Utilisation de préparations pour l'ingestion par voie orale, contenant, en des quantités de 0,001 µM à 10 µM (0,002 mg/kg à 2 mg/kg), au moins un alcamide choisi dans le groupe qui est constitué par le N-isobutylamide de l'acide 2E,4E-décadiénoïque (trans-pellitorine) ; le N-isobutylamide de l'acide 2E,4Z-décadiénoïque (cis-pellitorine) ; le N-isobutylamide de l'acide 2Z,4Z-décadiénoïque ; le N-isobutylamide de l'acide 2Z,4E-décadiénoïque ; le N-([2S]-2-méthylbutyl)-amide de l'acide 2E,4E-décadiénoïque ; le N-([2S]-2-méthylbutyl)-amide de l'acide 2E,4E-décadiénoïque ; le N-([2R]-2-méthylbutyl)-amide de l'acide 2E,4E-décadiénoïque ; le N-(2-méthylbutyl)amide de l'acide 2E,4Z-décadiénoïque ; le N-pipéride de l'acide 2E,4E-décadiénoïque (amide d'Achille) ; le N-isobutylamide de l'acide 2E-décénoïque ; le N-isobutylamide de l'acide 2E,6Z,8E-décatriénoïque (spilanthol) ; le N-([2S]-2-méthylbutyl)-amide de l'acide 2E,6Z,8E-décatriénoïque (homospilanthol) ; le N-([2R]-2-méthylbutyl)amide de l'acide 2E,6Z,8E-décatriénoïque; le N-isobutylamide de l'acide 2Z-décén-4-ynoïque ; le N-(2-méthylpropyl) amide de l'acide 2E,6Z,8E,10E-dodécatétraénoïque (α-sanshool) ; le N-(2-hydroxy-2-méthylpropyl)amide de l'acide 2E,6Z-8E,10E-dodécatétraénoïque (α-hydroxysanshool) ; le N-(2-hydroxy-2-méthylpropyl)amide de l'acide 2E,6E,8E,10E-dodécatétraénoïque (γ-hydroxysanshool) ; le N-(2-hydroxy-2-méthylpropyl)-amide de l'acide 2E,4E-8Z,10E,12E-tétradécapentaénoïque (γ-hydroxysanshool) ; le N-(2-hydroxy-2-méthylpropyl)-amide de l'acide 2E,4E,8E,10E,12E-tétradécapentaénoïque (γ-hydroxyisosanshool) ; le N-(2-méthyl-2-propényl)-amide de l'acide 2E,4E,8Z,10E,12E-tétradécapentaénoïque (y-déshydrosanshool) ; le N-(2-méthylpropyl)amide de l'acide 2E,4E,8Z,10E,12E-tétradécapentaénoïque (y-sanshool) ; le N-(2-hydroxy-2-méthylpropyl)amide de l'acide 2E,4E,8Z,11Z-tétradécatétraénoïque (bungeanool) ; le N-(2-hydroxy-2-méthylpropyl)amide de l'acide 2E,4E,8Z,11E-tétradécatétraénoïque (isobungeanool) ; le N-(2-hydroxy-2-méthylpropyl)amide de l'acide 2E,4E,8Z-tétradécatriénoïque (dihydrobungeanool) et le N-(2-hydroxy-2-méthylpropyl)amide de l'acide 2E,4E-tétradécadiénoïque (tétrahydrobungeanool) comme agents à ingérer par voie orale pour diminuer l'appétit.

12. Utilisation de préparations telles que définies dans la revendication 11, comme agents à ingérer par voie orale pour obtenir une sensation de satiété.

13. Utilisation de préparations telles que définies dans la revendication 11, comme agents à ingérer par voie orale pour diminuer la résorption calorique.
